# EUROPÄISCHE PATENTSCHRIFT

(11) **EP 4 362 852 B1**
(45) Veröffentlichungstag und Bekanntmachung des Hinweises auf die Patenterteilung: **28.08.2024**
(21) Anmeldenummer: 22738384.1
(22) Anmeldetag: 22.06.2022
(51) Int. Cl.: A61F 2/16

(54) **INJEKTOR MIT EINEM ERSTEN KOLBEN UND EINEM ZWEITEN KOLBEN**
INJECTOR COMPRISING A FIRST PISTON AND A SECOND PISTON
INJECTEUR COMPRENANT UN PREMIER PISTON ET UN SECOND PISTON

(30) Priorität: 28.06.2021 DE 102021116615
(43) Veröffentlichungstag der Anmeldung: 08.05.2024
(73) Patentinhaber: Carl Zeiss Meditec AG, 07745 Jena (DE)
(72) Erfinder: KELP, Martin, 73447 Oberkochen (DE); RINMAN, Alfred, 73447 Oberkochen (DE); MOEIN, Hadi, 73447 Oberkochen (DE); DAMM, Niklas, 73447 Oberkochen (DE)
(74) Vertreter: PATERIS Patentanwälte PartmbB
(86) Internationale Anmeldenummer: PCT/EP2022/066976
(87) Internationale Veröffentlichungsnummer: WO 2023/274800

(56) Entgegenhaltungen:
- EP-A1- 2 340 786
- WO-A1-00/40175
- US-A- 5 190 552

## Beschreibung

Die Erfindung betrifft einen Injektor für ein Einführen einer Intraokularlinse in den Kapselsack eines Auges.

Bei der Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges z. B. mittels Phakoemulsifikation zerkleinert und anschließend aus dem Kapselsack des Auges abgesaugt. Danach wird eine Intraokularlinse in das Auge eingesetzt. Dabei wird die Intraokularlinse gefaltet, so dass sie durch die Spitze des Injektors passt. Die Spitze wird durch den Schnitt in den Kapselsack eingeführt und die gefaltete Intraokularlinse wird von einem Kolben des Injektors durch die Spitze in den Kapselsack geschoben, in dem die Intraokularlinse sich entfaltet und somit die ursprüngliche Linse ersetzt. Der Injektor wird herkömmlich von Hand in den Kapselsack eingeführt und der Kolben wird oftmals von Hand angetrieben, um ihn zu verschieben. Wünschenswert wäre es daher, wenn der Injektor möglichst einfach bedienbar ist.

US 9 700 407 B2 offenbart einen Intraokularlinseninjektor. EP 2 340 786 A1 offenbart eine Vorrichtung zum Einführen einer Intraokularlinse. US 5 190 552 A offenbart einen Injektor, der mit einem geschlitzten Rohr gebildet ist.

Aufgabe der Erfindung ist es daher, einen Injektor für ein Einführen einer Intraokularlinse in den Kapselsack eines Auges zu schaffen, wobei der Injektor einfach bedienbar ist.

Der erfindungsgemäße Injektor für ein Einführen einer Intraokularlinse in den Kapselsack eines Auges weist einen Injektorkörper, der eine Injektorspitze mit einer Öffnung und ein der Öffnung abgewandtes proximales Ende aufweist, einen ersten Kolben, der zur Öffnung hin gerichtet ist und eingerichtet ist, die Intraokularlinse zu kontaktieren und zu verschieben, und einen zweiten Kolben auf, der im Bereich des proximalen Endes angeordnet ist und eingerichtet ist, von außerhalb des Injektors bedienbar zu sein, wobei der erste Kolben und der zweite Kolben jeweils längsverlagerbar entlang einer Injektorachse des Injektors in dem Injektorkörper angeordnet sind, sich entweder in einem Entkopplungszustand befinden, in dem die Kolben relativ zueinander längsverlagerbar sind, oder sich in einem Kopplungszustand befinden, in dem die Kolben starr in Richtung der Injektorachse miteinander gekoppelt sind und die Gesamtlängserstreckung der Kolben größer ist als im Entkopplungszustand. Ferner weist der erfindungsgemäße Injektor einen Griff auf, der in einem distalen Bereich des Injektorkörpers angeordnet ist, eingerichtet ist, von außerhalb des Injektors bedienbar zu sein und mit dem ersten Kolben gekoppelt ist, so dass, wenn die Kolben sich im Entkopplungszustand befinden, durch eine Bewegung des Griffs der erste Kolben zur Öffnung hin und relativ zum zweiten Kolben verlagerbar ist, damit die Kolben in den Kopplungszustand bringbar sind und mittels des ersten Kolbens durch die Kontaktierung der Intraokularlinse diese in der Injektorspitze zur Öffnung hin verschiebbar ist.

Sobald der erste Kolben und der zweite Kolben in dem Kopplungszustand sich befinden, ist der zweite Kolben zu bedienen, indem der zweite Kolben zur Öffnung hin längsverlagert wird. Durch die starre Kopplung der beiden Kolben im Kopplungszustand wird der erste Kolben entsprechend mitverlagert, wodurch die Intraokularlinse in der Injektorspitze zur Öffnung hin weiter verschoben wird. Sind beide Kolben in ihrer Endposition angelangt, ist die Intraokularlinse aus der Öffnung ausgetreten und kann somit in den Kapselsack eines Auges eingeführt werden.

Dadurch, dass das Verschieben der Intraokularlinse durch die beiden Kolben aufgeteilt ist, ist es vorteilhaft ermöglicht, den zweiten Kolben kürzer in seiner Längserstreckung auszuführen. Dementsprechend ist der Hub des zweiten Kolbens kürzer als der Hub bei einem herkömmlichen Injektor, bei dem beispielsweise nur ein einziger Kolben vorgesehen ist. Somit kann der erfindungsgemäße Injektor in seiner Längserstreckung kürzer ausgeführt werden, wodurch der erfindungsgemäße Injektor einfacher als der herkömmliche Injektor zu bedienen ist.

Sowohl der Griff als auch der zweite Kolben sind von außerhalb des Injektors bevorzugt manuell zu bedienen. Weil der Griff in dem distalen Bereich des Injektorkörpers und der zweite Kolben im Bereich des proximalen Endes des Injektorkörpers angeordnet sind, sind der Griff und der zweite Kolben von gegenüberliegenden Seiten her zu bedienen. Dadurch ist ein versehentliches Verwechseln des Griffs und des zweiten Kolbens bei deren Bedienung unwahrscheinlicher, als wären der Griff und der zweite Kolben etwa von derselben Seite des Injektors her zu bedienen. Dadurch ist der Injektor sicher und fehlerfrei bedienbar.

Der distale Bereich des Injektorkörpers kann dabei von einem ersten Abschnitt des Injektorkörpers gebildet sein, wobei der erste Abschnitt sich ausgehend von der Öffnung bis zu 50 % der Gesamtlänge des Injektorkörpers in Richtung der Injektorachse erstreckt. Der proximale Bereich des Injektorkörpers kann dabei von einem zweiten Abschnitt des Injektorkörpers gebildet sein, wobei der zweite Abschnitt sich ausgehend von dem proximalen Ende bis zu 50 % der Gesamtlänge des Injektorkörpers in Richtung der Injektorachse erstreckt.

Es ist bevorzugt, dass der Injektorkörper eine Injektorkörperaussparung aufweist, wobei der Griff sich durch die Injektorkörperaussparung in das Innere des Injektorkörpers erstreckt und unmittelbar mit dem ersten Kolben gekoppelt ist. Dabei ist besonders bevorzugt, dass der Griff lösbar mit dem ersten Kolben gekoppelt ist. Dadurch kann der Griff von dem verbliebenen Injektor entfernt werden, bevor die Injektorspitze in den Kapselsack des Auges eingeführt wird.

Dadurch weist der Injektor vorteilhaft an der Außenseite des Injektorkörpers keine störenden Bauteile auf, wenn die Injektorspitze in den Kapselsack eingeführt wird. Der Griff ist dazu vorgesehen, dann entfernt zu werden, wenn der erste Kolben und der zweite Kolben mittels des Griffs in den Kopplungszustand gebracht wurden. Es ist besonders bevorzugt, dass der erste Kolben eine Kolbennut aufweist, in die der Griff eingreift.

Alternativ ist es bevorzugt, dass der Injektor einen Schlitten aufweist, der längsverlagerbar an dem Injektorkörper angeordnet ist und unmittelbar mit dem ersten Kolben gekoppelt ist, wobei der Griff eingerichtet ist, wenn der Griff in Richtung weg von dem proximalen Ende verlagert wird, den Schlitten mitzunehmen, so dass der Griff mittelbar via den Schlitten mit dem ersten Kolben gekoppelt ist. Dabei ist besonders bevorzugt, dass der Schlitten einen Linsenhalter aufweist, der eingerichtet ist, die Intraokularlinse zu halten, wobei der Griff eingerichtet ist, wenn der Griff in Richtung weg von dem proximalen Ende verlagert wird und bevor der Griff den Schlitten mitnimmt, die Intraokularlinse aus dem Linsenhalter zu verlagern und zu falten. Dadurch ist der Griff sowohl eingerichtet, den ersten Kolben in Richtung zu der Öffnung hin zu verlagern, als auch eingerichtet, die Intraokularlinse zu falten.

Es ist besonders bevorzugt, dass der Schlitten lösbar mit dem ersten Kolben gekoppelt ist. Dadurch kann der Schlitten insbesondere zusammen mit dem Griff von dem verbliebenen Injektor entfernt werden, bevor die Injektorspitze in den Kapselsack des Auges eingeführt wird. Dadurch weist der Injektor an der Außenseite des Injektorkörpers keine störenden Bauteile auf, wenn die Injektorspitze in den Kapselsack eingeführt wird. Der Schlitten und optional der Griff sind dazu vorgesehen, dann entfernt zu werden, wenn der erste Kolben und der zweite Kolben mittels des Griffs in den Kopplungszustand gebracht wurden. Es ist bevorzugt, dass der erste Kolben eine Kolbennut aufweist, in die der Schlitten eingreift.

Es ist bevorzugt, dass der Griff eine Kappe aufweist, die einen Innenraum begrenzt, in dem der Injektorkörper angeordnet ist. Mittels der Kappe ist der Griff besonders einfach von einer Hand angreifbar, wodurch auch der erste Kolben besonders einfach in Richtung zu der Öffnung hin verlagert werden kann. Es ist besonders bevorzugt, dass die Kappe von dem Injektorkörper in Richtung der Injektorachse über die Öffnung hinaus absteht. Die Kappe weist besonders bevorzugt einen Mantel, der den Innenraum in Radialrichtung bezüglich der Injektorachse begrenzt, und eine Stirnwand auf, die in Richtung der Injektorachse neben dem Injektorkörper angeordnet ist und den Innenraum in Richtung der Injektorachse begrenzt. Dadurch ist der Injektorkörper in dem distalen Bereich vorteilhaft geschützt.

Der zweite Kolben weist bevorzugt an seiner der Öffnung zugewandten Stirnseite eine Kolbenöffnung auf, via die sich der erste Kolben in das Innere des zweiten Kolbens erstreckt und an der der erste Kolben in dem Entkopplungszustand längsverlagerbar relativ zu dem zweiten Kolben gelagert ist. Alternativ ist auch denkbar, dass der erste Kolben an seiner der Öffnung abgewandten Stirnseite eine Kolbenöffnung aufweist, via die sich der zweite Kolben in das Innere des ersten Kolbens erstreckt und an der der zweite Kolben in dem Entkopplungszustand längsverlagerbar relativ zu dem ersten Kolben gelagert ist. Bevorzugt ist es, dass die Kolben teleskopierend ausgebildet sind. Ferner ist es bevorzugt, dass die Kolben bezüglich der Injektorachse koaxial angeordnet sind.

Der zweite Kolben ist bevorzugt eingerichtet, durch eine Drückbewegung oder eine Schraubbewegung in Richtung zu der Öffnung hin längsverlagert zu werden. Besonders bevorzugt ist der zweite Kolben eingerichtet, dass die Drückbewegung oder die Schraubbewegung von Hand durchzuführen ist, d.h. der Injektor weist beispielsweise keinen Motor auf.

Es ist bevorzugt, dass der Injektor die Intraokularlinse aufweist. Die Intraokularlinse kann in dem Injektorkörper angeordnet sein. In dem Fall, dass der Linsenhalter vorgesehen ist, kann die Intraokularlinse alternativ in dem Linsenhalter angeordnet sein.

Es ist bevorzugt, dass der erste Kolben einen Kolbenvorsprung aufweist und der zweite Kolben eine Kolbenaussparung aufweist, wobei der Kolbenvorsprung eingerichtet ist, außerhalb der Kolbenaussparung angeordnet zu sein, wenn der erste Kolben und der zweite Kolben in dem Entkopplungszustand sind, und eingerichtet ist, zum Bringen des ersten Kolbens und des zweiten Kolbens in den Kopplungszustand in die Kolbenaussparung einzugreifen, wenn der erste Kolben in Richtung zu der Öffnung hin verlagert wird. Dies stellt eine besonders einfache Konstruktion bereit, mittels der der erste Kolben und der zweite Kolben in den Kopplungszustand gebracht werden können.

Im Folgenden wird anhand der beigefügten schematischen Zeichnungen die Erfindung näher erläutert. Es zeigen
Figur 1 eine Längsschnittdarstellung in der Draufsicht einer ersten Ausführungsform eines erfindungsgemäßen Injektors zu einem ersten Zeitpunkt,
Figur 2 die Längsschnittdarstellung aus Figur 1 zu einem zweiten Zeitpunkt,
Figur 3 die Längsschnittdarstellung aus Figur 1 zu einem dritten Zeitpunkt,
Figur 4 eine Längsschnittdarstellung in der Draufsicht durch eine zweite Ausführungsform des erfindungsgemäßen Injektors zu einem ersten Zeitpunkt,
Figur 5 die Längsschnittdarstellung aus Figur 4 zu einem zweiten Zeitpunkt,
Figur 6 eine Längsschnittdarstellung in der Draufsicht einer dritten Ausführungsform des erfindungsgemäßen Injektors zu einem ersten Zeitpunkt, wobei nicht schraffierte Bauteile des Injektors ungeschnitten dargestellt sind,
Figur 7 eine Längsschnittdarstellung in der Seitenansicht der dritten Ausführungsform zu dem ersten Zeitpunkt, so wie in Figur 6 gezeigt, wobei nicht schraffierte Bauteile des Injektors ungeschnitten dargestellt sind,
Figur 8 die Längsschnittdarstellung aus Figur 6 zu einem zweiten Zeitpunkt,
Figur 9 die Längsschnittdarstellung aus Figur 6 zu einem dritten Zeitpunkt,
Figur 10 die Längsschnittdarstellung aus Figur 6 zu einem vierten Zeitpunkt,
Figur 11 eine Darstellung eines Details I aus Figur 1, wobei ein erster Kolben und ein zweiter Kolben des Injektors sich in einem Entkopplungszustand befinden, und
Figur 12 eine Darstellung eines Details II aus Figur 2, wobei der erste Kolben und der zweite Kolben sich in einem Kopplungszustand befinden.

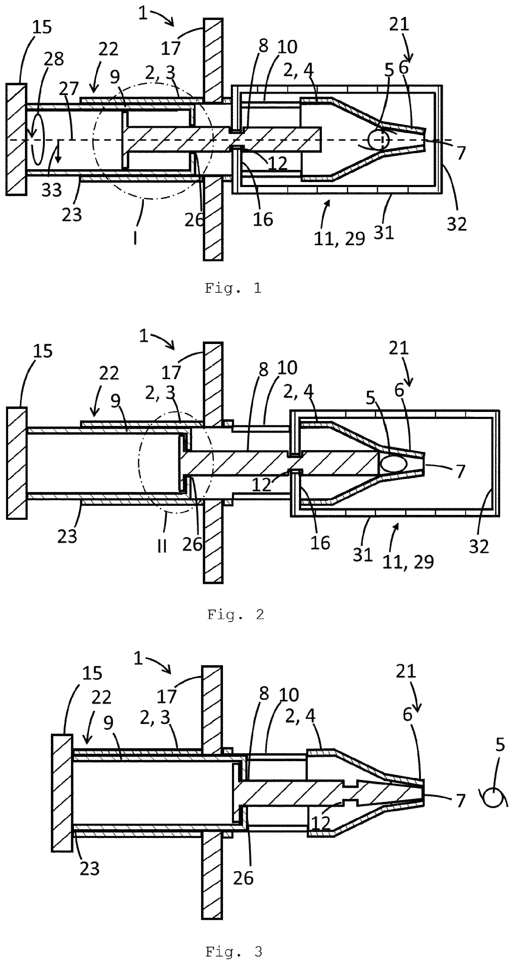

Wie es aus Figuren 1 bis 12 ersichtlich ist, weist ein Injektor 1 für ein Einführen einer Intraokularlinse 5 in den Kapselsack eines Auges einen Injektorkörper 2, der eine Injektorspitze 6 mit einer Öffnung 7 und ein der Öffnung 7 abgewandtes proximales Ende 23 aufweist, einen ersten Kolben 8, der zur Öffnung 7 hin gerichtet ist und eingerichtet ist, die Intraokularlinse 5 zu kontaktieren und zu verschieben, und einen zweiten Kolben 9 auf, der im Bereich des proximalen Endes 23 angeordnet ist und eingerichtet ist, von außerhalb des Injektors 1 bedienbar zu sein. Der erste Kolben 8 und der zweite Kolben 9 sind jeweils längsverlagerbar entlang einer Injektorachse 27 des Injektors 1 in dem Injektorkörper 2 angeordnet, befinden sich entweder in einem Entkopplungszustand, in dem die Kolben 8, 9 relativ zueinander längsverlagerbar sind, oder in einem Kopplungszustand, in dem die Kolben 8, 9 starr in Richtung der Injektorachse 27 miteinander gekoppelt sind und die Gesamtlängserstreckung der Kolben 8, 9 größer ist als im Entkopplungszustand. Zudem weist der Injektor 1 einen Griff 29 auf, der in einem distalen Bereich 21 des Injektorkörpers 2 angeordnet ist, eingerichtet ist, von außerhalb des Injektors 1 bedienbar zu sein und mit dem ersten Kolben 8 gekoppelt ist, so dass, wenn die Kolben 8, 9 sich im Entkopplungszustand befinden, durch eine Bewegung des Griffs 29 der erste Kolben 8 zur Öffnung 7 hin und relativ zum zweiten Kolben 9 verlagerbar ist, damit die Kolben 8, 9 in den Kopplungszustand bringbar sind und mittels des ersten Kolbens 8 durch die Kontaktierung der Intraokularlinse 5 diese in der Injektorspitze 6 zur Öffnung 7 hin verschiebbar ist.

Der distale Bereich 21 des Injektorkörpers 2 kann dabei von einem ersten Abschnitt des Injektorkörpers 2 gebildet sein, wobei der erste Abschnitt sich ausgehend von der Öffnung 7 bis zu 50 % der Gesamtlänge des Injektorkörpers 2 in Richtung der Injektorachse 27 erstreckt. Der proximale Bereich 22 des Injektorkörpers 2 kann dabei von einem zweiten Abschnitt des Injektorkörpers 2 gebildet sein, wobei der zweite Abschnitt sich ausgehend von dem proximalen Ende 23 bis zu 50 % der Gesamtlänge des Injektorkörpers 2 in Richtung der Injektorachse 27 erstreckt.

Figuren 1 bis 6 und 8 bis 10 zeigen zudem, dass der Injektor 1 einen Injektorgriff 17 aufweisen kann, der an dem Injektorkörper 2 angebracht ist, nach außen von dem Injektorkörper 2 absteht und in Richtung der Injektorachse 27 zwischen dem proximalen Ende 23 und dem Griff 29 angeordnet ist.

Figuren 1, 2, 4 und 6 bis 9 zeigen, dass der Griff 29 eine Kappe 11 aufweisen kann, die einen Innenraum begrenzt, in dem der Injektorkörper 2 angeordnet ist. Die Kappe 11 kann von dem Injektorkörper 2 in Richtung der Injektorachse 27 über die Öffnung 7 hinaus abstehen. Die Kappe 11 kann einen Mantel 31 aufweisen, der den Innenraum in Radialrichtung 33 bezüglich der Injektorachse 27 begrenzt und insbesondere in der Umfangsrichtung 28 den Innenraum vollständig begrenzt, d.h. der Mantel 31 ist in der Umfangsrichtung 28 ohne Unterbrechungen ausgeführt. Die Umfangsrichtung 28 und die Radialrichtung 33 sind beispielhaft in Figur 1 eingezeichnet. Zudem kann die Kappe 11 eine Stirnwand 32 aufweisen, die in Richtung der Injektorachse 27 neben dem Injektorkörper 2 angeordnet ist und den Innenraum in Richtung der Injektorachse 27 begrenzt. Die Kappe 11 kann lediglich eine einzige Kappenöffnung aufweisen, die der Stirnwand 32 abgewandt angeordnet ist.

Wie es aus Figuren 1 bis 12 ersichtlich ist, kann der zweite Kolben 9 an seiner der Öffnung 7 zugewandten Stirnseite eine Kolbenöffnung 26 aufweisen, via die sich der erste Kolben 8 in das Innere des zweiten Kolbens 9 erstreckt und an der der erste Kolben 8 in dem Entkopplungszustand längsverlagerbar relativ zu dem zweiten Kolben 9 gelagert ist.

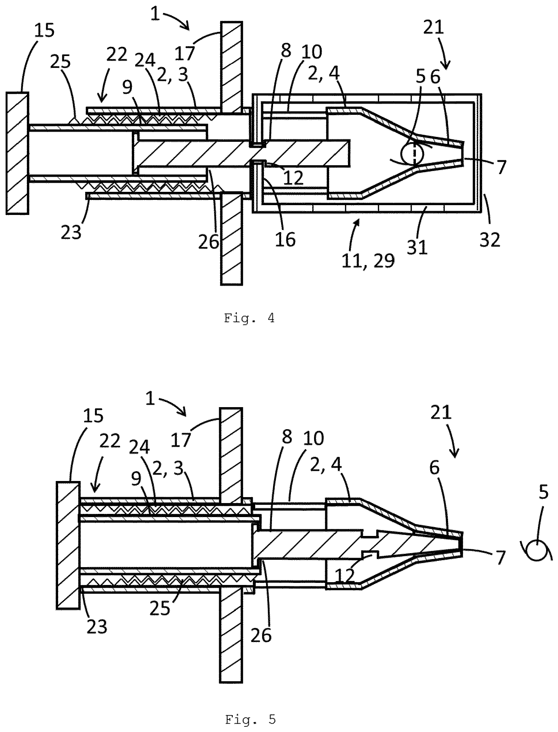

Bei der ersten Ausführungsform des erfindungsgemäßen Injektors 1, die in Figuren 1 bis 3 dargestellt ist, und der zweiten Ausführungsform des Injektors 1, die in Figuren 4 und 5 dargestellt ist, weist der Injektorkörper 2 eine Injektorkörperaussparung 10 auf, wobei der Griff 29 sich via die Injektorkörperaussparung 10 in das Innere des Injektorkörpers 2 erstreckt und unmittelbar mit dem ersten Kolben 8 gekoppelt ist. In dem Fall, dass der Griff 29 die Kappe 11 mit dem Mantel 31 aufweist, kann die Kappe 11 einen Kappenvorsprung 16 (vergleiche Figuren 1, 2 und 4) aufweisen, der ein Teil des Griffs 29 ist, radial nach innen von dem Mantel 31 absteht und mit dem ersten Kolben 8 in Eingriff steht. Der Griff 29 kann lösbar mit dem ersten Kolben 8 gekoppelt sein. Dazu kann der erste Kolben 8 eine Kolbennut 12 aufweisen, in die der Griff 29 eingreift.

In Figuren 1 und 4 ist zu dem ersten Zeitpunkt der ersten Ausführungsform und dem ersten Zeitpunkt der zweiten Ausführungsform der Injektor 1 in dem Entkopplungszustand des ersten Kolbens 8 und des zweiten Kolbens 9 dargestellt. Indem der Griff 29 nun von dem proximalen Ende 23 weg verlagert wird, beispielsweise indem mit einer Hand der Injektorgriff 17, mit einer anderen Hand der Griff 29 angefasst werden und beide Hände voneinander wegbewegt werden, können der erste Kolben 8 und der zweite Kolben 9 in den Kopplungszustand gebracht werden, was zu dem zweiten Zeitpunkt der ersten Ausführungsform gemäß Figur 2 dargestellt ist. Dabei kann auch die Intraokularlinse 5 in Richtung zu der Öffnung 7 hin verlagert werden. Figuren 1 bis 5 zeigen, dass die Injektorkörperaussparung 10 ein distales Ende aufweisen kann, dass von dem Injektorkörper 2 gebildet ist und an das der Griff 29 anschlagen kann (vergleiche Figur 2), wenn der Griff 29 weg von dem proximalen Ende 23 verlagert wird. Wird der Griff 29 dann weiter weg von dem proximalen Ende 23 verlagert, kann derjenige Teil des Griffs 29, der in der Kolbennut 12 angeordnet ist, in Richtung zu dem proximalen Ende 23 verbiegen und dadurch außer Eingriff mit der Kolbennut 12 gelangen. Dadurch ist der Griff 29 nicht mehr mit dem ersten Kolben 8 gekoppelt und kann daher von dem Injektorkörper 2 entfernt werden. Dies ist zu dem dritten Zeitpunkt der ersten Ausführungsform (siehe Figur 3) und dem zweiten Zeitpunkt der zweiten Ausführungsform (siehe Figur 5) dargestellt.

Zu dem dritten Zeitpunkt der ersten Ausführungsform, vergleiche Figur 3, und dem zweiten Zeitpunkt der zweiten Ausführungsform, vergleiche Figur 5, wurde in dem Kopplungszustand der zweite Kolben 9 in Richtung der Öffnung 7 hin verlagert, wodurch auch der erste Kolben 8 in Richtung der Öffnung 7 hin verlagert wurde. Dabei hat der erste Kolben 8 die Intraokularlinse 5 via die Öffnung 7 aus dem Injektor heraus verlagert. Der zweite Kolben 9 kann, wie es beispielhaft für die erste Ausführungsform dargestellt ist, eingerichtet sein, durch eine Drückbewegung, insbesondere von Hand und ohne einen Motor, in Richtung zu der Öffnung 7 hin längsverlagert werden. Alternativ kann der zweite Kolben 9, wie es beispielhaft für die zweite Ausführungsform dargestellt ist, eingerichtet sein, durch eine Schraubbewegung, insbesondere von Hand und ohne einen Motor, in Richtung zu der Öffnung 7 hin längsverlagert zu werden. Dazu kann der Injektorkörper 2 ein Innengewinde 24 aufweisen und der zweite Kolben 9 kann ein Außengewinde 25 aufweisen, wobei das Innengewinde 24 mit dem Außengewinde 25 in Eingriff steht.

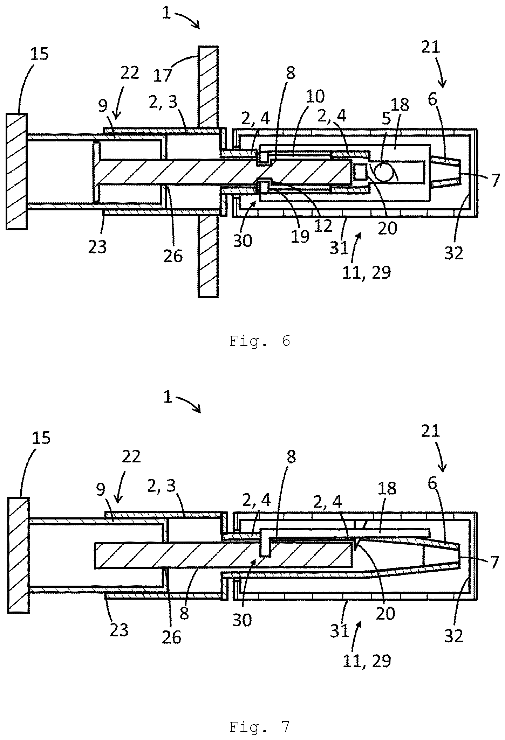

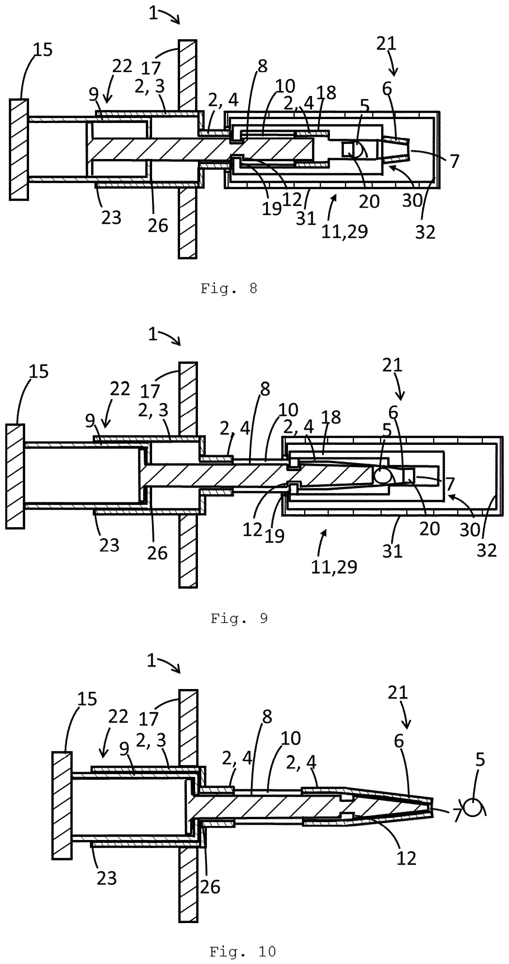

Figuren 6 bis 10 zeigen, dass der Injektor 1 gemäß der dritten Ausführungsform einen Schlitten 30 aufweisen kann, der längsverlagerbar an dem Injektorkörper 2 angeordnet ist und unmittelbar mit dem ersten Kolben 8 gekoppelt ist, wobei der Griff 29 eingerichtet ist, wenn der Griff 29 in Richtung weg von dem proximalen Ende 23 verlagert wird, den Schlitten 30 mitzunehmen, so dass der Griff 29 mittelbar via den Schlitten 30 mit dem ersten Kolben 8 gekoppelt ist. Der Schlitten 30 kann einen Linsenhalter 18 aufweisen, der eingerichtet ist, die Intraokularlinse 5 zu halten, wobei der Griff 29 eingerichtet ist, wenn der Griff 29 in Richtung weg von dem proximalen Ende 23 verlagert wird und bevor der Griff 29 den Schlitten 30 mitnimmt, die Intraokularlinse 5 aus dem Linsenhalter 18 zu verlagern und zu falten. Dazu kann der Griff 29 ein Faltelement 20 aufweisen, das von dem verbliebenen Griff 29 absteht, in den Injektorkörper 2 hineinragt und eingerichtet ist, die Intraokularlinse 5 aus dem Linsenhalter 18 zu verlagern und zu falten. Der Schlitten 30 und das Faltelement 20 sind in Figuren 6 bis 10 in Draufsicht dargestellt, während alle anderen Bauteile des Injektors 1 im Längsschnitt dargestellt sind. In Figuren 6 und 7 ist der Injektor 1 zu einem ersten Zeitpunkt dargestellt, zu dem die Intraokularlinse 5 in dem Linsenhalter 18 angeordnet ist und der erste Kolben 8 und der zweite Kolben 9 in dem Entkopplungszustand sind. Indem der Griff 29 nun von dem proximalen Ende 23 weg verlagert wird, beispielsweise indem mit einer Hand der Injektorgriff 17, mit einer anderen Hand der Griff 29 angefasst werden und beide Hände voneinander wegbewegt werden, verlagert sich der Griff 29 so lange, bis der Griff 29 zu einem in Figur 8 dargestellten zweiten Zeitpunkt an den Schlitten 30 anschlägt. Wie es aus Figur 8 ersichtlich ist, wurde die Intraokularlinse 5 aus dem Linsenhalter 18 heraus verlagert und befindet sich unterhalb des Faltelements 20.

Wird nun der Griff 29 weiter weg von dem proximalen Ende 23 verlagert, nimmt der Griff 29, dadurch dass der Griff 29 an den Schlitten 30 angeschlagen ist, den Schlitten 30 mit. Dabei wird auch der erste Kolben 8 in Richtung zu der Öffnung 7 hin verlagert, wobei der Kolben 8 dabei die Intraokularlinse 5 kontaktiert und in Richtung zu der Öffnung 7 hin verlagert. Figuren 6 bis 10 zeigen, dass die Injektorkörperaussparung 10 ein distales Ende aufweisen kann, dass von dem Injektorkörper 2 gebildet ist und an das der Schlitten 30 anschlagen kann (vergleiche Figur 9, die den Injektor 1 zu einem dritten Zeitpunkt zeigt), wenn der Griff 29 weg von dem proximalen Ende 23 verlagert wird.

Figuren 5 bis 9 zeigen, dass der Schlitten 30 lösbar mit dem ersten Kolben 8 gekoppelt sein kann. Dazu kann der erste Kolben 8 eine Kolbennut 12 aufweisen, in die der Schlitten 30 eingreift. Insbesondere kann der Schlitten 30 einen Schlittenzapfen 19 aufweisen, der in die Kolbennut 12 eingreift. Wird der Griff 29 nach dem dritten Zeitpunkt weiter weg von dem proximalen Ende 23 verlagert, kann derjenige Teil des Schlittens 30, der an und in der Kolbennut 12 angeordnet ist, in Richtung zu dem proximalen Ende 23 sich verformen und dadurch außer Eingriff mit der Kolbennut 12 gelangen. Dadurch ist der Schlitten 30 nicht mehr mit dem ersten Kolben 8 gekoppelt und kann daher zusammen mit dem Griff 29 von dem Injektorkörper 2 entfernt werden. Dies ist zu dem vierten Zeitpunkt (siehe Figur 10) dargestellt.

Zu dem vierten Zeitpunkt, vergleiche Figur 10, wurde in dem Kopplungszustand der zweite Kolben 9 in Richtung der Öffnung 7 hin verlagert, wodurch auch der erste Kolben 8 in Richtung der Öffnung 7 hin verlagert wurde. Dabei hat der erste Kolben 8 die Intraokularlinse 5 via die Öffnung 7 aus dem Injektor heraus verlagert. Der zweite Kolben 9 kann eingerichtet sein, durch eine Drückbewegung, wie es auch für die dritte Ausführungsform dargestellt ist, oder eine Schraubbewegung in Richtung zu der Öffnung 7 hin, längsverlagert zu werden.

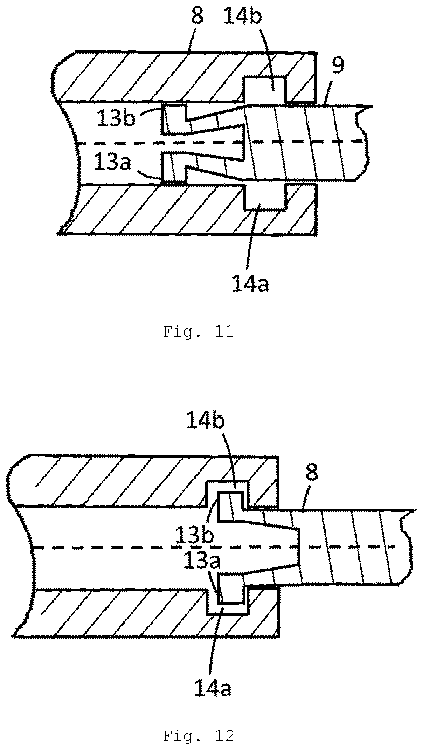

Figuren 11 und 12 zeigen, dass der erste Kolben 8 einen Kolbenvorsprung 13a aufweisen kann und der zweite Kolben 9 eine Kolbenaussparung 14a aufweisen kann, wobei der Kolbenvorsprung 13a eingerichtet ist, außerhalb der Kolbenaussparung 14a angeordnet zu sein, wenn der erste Kolben 8 und der zweite Kolben 9 in dem Entkopplungszustand sind (siehe Figur 11), und eingerichtet ist, zum Bringen des ersten Kolbens 8 und des zweiten Kolbens 9 in den Kopplungszustand in die Kolbenaussparung 14a einzugreifen, (siehe Figur 12) wenn der erste Kolben 8 in Richtung zu der Öffnung 7 hin verlagert wird. Die Kolbenaussparung 14a kann in die in der Radialrichtung 33 innenliegende Seite des zweiten Kolbens 9 eingebracht sein. Der Kolbenvorsprung 13a kann an die in der Radialrichtung 33 innenliegende Seite des zweiten Kolbens 9 anstoßen, wenn der Kolbenvorsprung 13a außerhalb der Kolbenaussparung 14a angeordnet ist, und dadurch in der Radialrichtung 33 nach innen gedrückt und somit gespannt sein. Wenn der erste Kolben 8 in Richtung zu der Öffnung 7 hin verlagert wird, gelangt der Kolbenvorsprung 13a in die Kolbenaussparung 14a, wobei der Kolbenvorsprung 13a dadurch zumindest weniger gespannt ist. Figuren 11 und 12 zeigen zudem, dass der erste Kolben 8 einen weiteren Kolbenvorsprung 13b aufweisen kann und der zweite Kolben 9 eine weitere Kolbenaussparung 14b aufweisen kann, wobei der weitere Kolbenvorsprung 13b eingerichtet ist, außerhalb der weiteren Kolbenaussparung 14b angeordnet zu sein, wenn der erste Kolben 8 und der zweite Kolben 9 in dem Entkopplungszustand sind (siehe Figur 11), und eingerichtet ist, zum Bringen des ersten Kolbens 8 und des zweiten Kolbens 9 in den Kopplungszustand in die weitere Kolbenaussparung 14b einzugreifen (siehe Figur 12), wenn der erste Kolben 8 in Richtung zu der Öffnung 7 hin verlagert wird.

Figuren 1 bis 10 zeigen, dass der Injektor 1 die Intraokularlinse 5 aufweisen kann. Die Intraokularlinse 5 kann zu dem jeweils ersten Zeitpunkt in dem Injektorkörper 2 und/oder in dem Linsenhalter 18 angeordnet sein.

Wie es aus Figuren 1 bis 10 ersichtlich ist, kann der zweite Kolben 9 an seinem distalen Ende einen Kolbenkopf 15 aufweisen. Der Kolbenkopf 15 kann eingerichtet sein, an den Injektorkörper 2 anzuschlagen, um die Bewegung des zweiten Kolbens in Richtung zu der Öffnung 7 hin zu begrenzen.

Figuren 6 bis 10 zeigen, dass der Injektorkörper 2 einen Zylinder 3, in dem der zweite Kolben 9 längsverlagerbar gelagert ist, und eine Kartusche 4 aufweisen kann, in der der erste Kolben 9 zum Längsverlagern der Intraokularlinse 5 angeordnet ist. Der Zylinder 3 und die Kartusche 4 können lösbar miteinander gekoppelt sein.

### Bezugszeichenliste

1 Injektor
2 Injektorkörper
3 Zylinder
4 Kartusche
5 Intraokularlinse
6 Injektorspitze
7 Öffnung
8 erster Kolben
9 zweiter Kolben
10 Injektorkörperaussparung
11 Kappe
12 Kolbennut
13a Kolbenvorsprung
13b weiterer Kolbenvorsprung
14a Kolbenaussparung
14b weitere Kolbenaussparung
15 Kolbenkopf
16 Kappenvorsprung
17 Injektorgriff
18 Linsenhalter
19 Schlittenzapfen
20 Faltelement
21 distaler Bereich
22 proximaler Bereich
23 proximales Ende
24 Innengewinde
25 Außengewinde
26 Kolbenöffnung
27 Injektorachse
28 Umfangsrichtung
29 Griff
30 Schlitten
31 Mantel
32 Stirnwand
33 Radialrichtung

## Patentansprüche

1. Injektor für ein Einführen einer Intraokularlinse (5) in den Kapselsack eines Auges, mit einem Injektorkörper (2), der eine Injektorspitze (6) mit einer Öffnung (7) und ein der Öffnung (7) abgewandtes proximales Ende (23) aufweist, einem ersten Kolben (8), der zur Öffnung (7) hin gerichtet ist und eingerichtet ist, die Intraokularlinse (5) zu kontaktieren und zu verschieben, einem zweiten Kolben (9), der eingerichtet ist, von außerhalb des Injektors (1) bedienbar zu sein, wobei der erste Kolben (8) und der zweite Kolben (9) jeweils längsverlagerbar entlang einer Injektorachse (27) des Injektors (1) in dem Injektorkörper (2) angeordnet sind, und einem Griff (29), der in einem distalen Bereich (21) des Injektorkörpers (2) angeordnet ist, eingerichtet ist, von außerhalb des Injektors (1) bedienbar zu sein und mit dem ersten Kolben (8) gekoppelt ist, **dadurch gekennzeichnet, dass** der zweite Kolben (9) im Bereich des proximalen Endes (23) angeordnet ist, wobei der erste Kolben (8) und der zweite Kolben (9) sich entweder in einem Entkopplungszustand befinden, in dem die Kolben (8, 9) relativ zueinander längsverlagerbar sind, oder sich in einem Kopplungszustand befinden, in dem die Kolben (8, 9) starr in Richtung der Injektorachse (27) miteinander gekoppelt sind und die Gesamtlängserstreckung der Kolben (8, 9) größer ist als im Entkopplungszustand, wobei der Griff (29) mit dem ersten Kolben (8) so gekoppelt ist, dass, wenn die Kolben (8, 9) sich im Entkopplungszustand befinden, durch eine Bewegung des Griffs (29) der erste Kolben (8) zur Öffnung (7) hin und relativ zum zweiten Kolben (9) verlagerbar ist, damit die Kolben (8, 9) in den Kopplungszustand bringbar sind und mittels des ersten Kolbens (8) durch die Kontaktierung der Intraokularlinse (5) diese in der Injektorspitze (6) zur Öffnung (7) hin verschiebbar ist.

2. Injektor gemäß Anspruch 1, wobei der Injektorkörper (2) eine Injektorkörperaussparung (10) aufweist, wobei der Griff (29) sich durch die Injektorkörperaussparung (10) in das Innere des Injektorkörpers (2) erstreckt und unmittelbar mit dem ersten Kolben (8) gekoppelt ist.

3. Injektor gemäß Anspruch 2, wobei der Griff (29) lösbar mit dem ersten Kolben (8) gekoppelt ist.

4. Injektor gemäß Anspruch 1, wobei der Injektor (1) einen Schlitten (30) aufweist, der längsverlagerbar an dem Injektorkörper (2) angeordnet ist und unmittelbar mit dem ersten Kolben (8) gekoppelt ist, wobei der Griff (29) eingerichtet ist, wenn der Griff (29) in Richtung weg von dem proximalen Ende (23) verlagert wird, den Schlitten (30) mitzunehmen, so dass der Griff (29) mittelbar via den Schlitten (30) mit dem ersten Kolben (8) gekoppelt ist.

5. Injektor gemäß Anspruch 4, wobei der Schlitten (30) einen Linsenhalter (18) aufweist, der eingerichtet ist, die Intraokularlinse (5) zu halten, wobei der Griff (29) eingerichtet ist, wenn der Griff (29) in Richtung weg von dem proximalen Ende (23) verlagert wird und bevor der Griff (29) den Schlitten (30) mitnimmt, die Intraokularlinse (5) aus dem Linsenhalter (18) zu verlagern und zu falten.

6. Injektor gemäß Anspruch 4 oder 5, wobei der Schlitten (30) lösbar mit dem ersten Kolben (8) gekoppelt ist.

7. Injektor gemäß einem der Ansprüche 1 bis 6, wobei der Griff (29) eine Kappe (11) aufweist, die einen Innenraum begrenzt, in dem der Injektorkörper (2) angeordnet ist.

8. Injektor gemäß Anspruch 7, wobei die Kappe (11) von dem Injektorkörper (2) in Richtung der Injektorachse (27) über die Öffnung (7) hinaus absteht.

9. Injektor gemäß Anspruch 8, wobei die Kappe (11) einen Mantel (31), der den Innenraum in Radialrichtung (33) bezüglich der Injektorachse (27) begrenzt, und eine Stirnwand (32) aufweist, die in Richtung der Injektorachse (27) neben dem Injektorkörper (2) angeordnet ist und den Innenraum in Richtung der Injektorachse (27) begrenzt.

10. Injektor gemäß einem der Ansprüche 1 bis 9, wobei der zweite Kolben (9) an seiner der Öffnung (7) zugewandten Stirnseite eine Kolbenöffnung (26) aufweist, via die sich der erste Kolben (8) in das Innere des zweiten Kolbens (9) erstreckt und an der der erste Kolben (8) in dem Entkopplungszustand längsverlagerbar relativ zu dem zweiten Kolben (9) gelagert ist.

## Claims

1. Injector for inserting an intraocular lens (5) into the capsular bag of an eye, comprising an injector body (2) having an injector tip (6) with an opening (7) and a proximal end (23) facing away from the opening (7); a first piston (8) which is directed toward the opening (7) and specified to contact and displace the intraocular lens (5); a second piston (9) which is specified to be able to be operated from outside the injector (1), wherein the first piston (8) and the second piston (9) are each disposed in the injector body (2) so as to be longitudinally displaceable along an injector axis (27) of the injector (1); and a handle (29) which is disposed in a distal region (21) of the injector body (2), specified to be able to be operated from outside the injector (1) and coupled to the first piston (8), **characterized in that** the second piston (9) is disposed in the region of the proximal end (23), wherein the first piston (8) and the second piston (9) are either in a decoupling state in which the pistons (8, 9) can be displaced longitudinally relative to each other or in a coupling state in which the pistons (8, 9) are rigidly coupled together in the direction of the injector axis (27) and the total longitudinal extent of the pistons (8, 9) is larger than in the decoupling state, wherein the handle (29) is coupled to the first piston (8) so that when the pistons (8, 9) are in the decoupling state, the first piston (8) is displaceable toward the opening (7) and relative to the second piston (9) by moving the handle (29) such that the pistons (8, 9) can be brought into the coupling state and, by way of contact between the first piston (8) and the intraocular lens (5), the latter is able to be shifted in the injector tip (6) toward the opening (7).

2. Injector according to Claim 1, wherein the injector body (2) has an injector body clearance (10), wherein the handle (29) extends through the injector body clearance (10) into the interior of the injector body (2) and is directly coupled to the first piston (8).

3. Injector according to Claim 2, wherein the handle (29) is releasably coupled to the first piston (8).

4. Injector according to Claim 1, wherein the injector (1) has a slide (30) which is disposed longitudinally displaceable on the injector body (2) and is directly coupled to the first piston (8), wherein when the handle (29) is displaced away from the proximal end (23), the handle (29) is specified to entrain the slide (30) so that the handle (29) is indirectly coupled to the first piston (8) by way of the slide (30).

5. Injector according to Claim 4, wherein the slide (30) has a lens holder (18), which is specified to hold the intraocular lens (5), wherein when the handle (29) is displaced away from the proximal end (23) and before the handle (29) entrains the slide (30), the handle (29) is specified to displace the intraocular lens (5) out of the lens holder (18) and fold said intraocular lens (5).

6. Injector according to Claim 4 or 5, wherein the slide (30) is releasably coupled to the first piston (8).

7. Injector according to one of Claims 1 to 6, wherein the handle (29) has a cap (11) which delimits an interior space in which the injector body (2) is disposed.

8. Injector according to Claim 7, wherein the cap (11) projects from the injector body (2) beyond the opening (7) in the direction of the injector axis (27).

9. Injector according to Claim 8, wherein the cap (11) has a casing (31), which delimits the interior space in the radial direction (33) with respect to the injector axis (27), and an end wall (32) which in the direction of the injector axis (27) is disposed next to the injector body (2) and delimits the interior space in the direction of the injector axis (27).

10. Injector according to one of Claims 1 to 9, wherein the second piston (9) on the end side thereof facing the opening (7) has a piston opening (26) by way of which the first piston (8) extends into the interior of the second piston (9) and on which the first piston (8) in the decoupling state is mounted so as to be longitudinally displaceable relative to the second piston (9).

## Revendications

1. Injecteur pour une introduction d'une lentille intraoculaire (5) dans le sac capsulaire d'un oeil, comprenant un corps d'injecteur (2) qui présente une pointe d'injecteur (6) avec une ouverture (7) et une extrémité proximale (23) opposée à l'ouverture (7), un premier piston (8) qui est dirigé vers l'ouverture (7) et qui est conçu pour entrer en contact avec la lentille intraoculaire (5) et la déplacer, un deuxième piston (9) qui est conçu de façon à être actionné depuis l'extérieur de l'injecteur (1), le premier piston (8) et le deuxième piston (9) étant respectivement agencés dans le corps d'injecteur (2) de manière à pouvoir être déplacés longitudinalement le long d'un axe d'injecteur (27) de l'injecteur (1), et une poignée (29), agencée dans une zone distale (21) du corps d'injecteur (2), qui est agencée de manière à pouvoir être actionnée depuis l'extérieur de l'injecteur (1) et qui est reliée au premier piston (8), **caractérisé, en ce que** le deuxième piston (9) est agencé dans la zone de l'extrémité proximale (23), le premier piston (8) et le deuxième piston (9) se trouvant soit dans un état de découplage dans lequel les pistons (8, 9) sont aptes à être déplacés longitudinalement l'un par rapport à l'autre, soit dans un état de couplage dans lequel les pistons (8, 9) sont reliés rigidement l'un à l'autre dans la direction de l'axe d'injecteur (27) et l'extension longitudinale totale des pistons (8, 9) est plus grande que dans l'état de découplage, la poignée (29) étant reliée au premier piston (8) de telle sorte que, lorsque les pistons (8, 9) sont dans l'état de découplage, un mouvement de la poignée (29) permet de déplacer le premier piston (8) vers l'ouverture (7) et par rapport au deuxième piston (9), de sorte que les pistons (8, 9) sont aptes à être amenés à l'état de couplage et qu'au moyen du premier piston (8), par la venue en contact avec la lentille intraoculaire (5), celle-ci est apte à être déplacée dans la pointe de l'injecteur (6) vers l'ouverture (7).

2. Injecteur selon la revendication 1, dans lequel le corps d'injecteur (2) présente un évidement (10) de corps d'injecteur, la poignée (29) s'étendant à travers l'évidement (10) de corps d'injecteur à l'intérieur du corps d'injecteur (2) et étant directement reliée au premier piston (8).

3. Injecteur selon la revendication 2, dans lequel la poignée (29) est reliée de manière amovible au premier piston (8).

4. Injecteur selon la revendication 1, dans lequel l'injecteur (1) comprend un coulisseau (30) monté mobile longitudinalement sur le corps d'injecteur (2) et directement relié au premier piston (8), la poignée (29) étant agencée, lorsque la poignée (29) est déplacée de façon à s'éloigner de l'extrémité proximale (23), pour entraîner le coulisseau (30), de sorte que la poignée (29) est reliée indirectement au premier piston (8) via le coulisseau (30).

5. Injecteur selon la revendication 4, dans lequel le coulisseau (30) comprend un porte-lentille (18) agencé pour maintenir la lentille intraoculaire (5), la poignée (29) étant agencée pour, lorsque la poignée (29) est déplacée de façon à s'éloigner de l'extrémité proximale (23) et avant que la poignée (29) n'entraîne le coulisseau (30), déplacer et déplier la lentille intraoculaire (5) hors du porte-lentille (18).

6. Injecteur selon la revendication 4 ou la revendication 5, dans lequel le coulisseau (30) est couplé de manière amovible au premier piston (8).

7. Injecteur selon l'une des revendications 1 à 6, dans lequel la poignée (29) comporte un capuchon (11) délimitant un espace intérieur dans lequel est agencé le corps d'injecteur (2).

8. Injecteur selon la revendication 7, dans lequel le capuchon (11) fait saillie du corps d'injecteur (2) selon la direction de l'axe d'injecteur (27) au-delà de l'ouverture (7).

9. Injecteur selon la revendication 8, dans lequel le capuchon (11) comporte une enveloppe (31) délimitant l'espace intérieur dans la direction radiale (33) par rapport à l'axe (27) de l'injecteur, et une paroi frontale (32) agencée à côté du corps (2) de l'injecteur dans la direction de l'axe (27) de l'injecteur et délimitant l'espace intérieur dans la direction de l'axe (27) de l'injecteur.

10. Injecteur selon l'une des revendications 1 à 9, dans lequel le deuxième piston (9) présente sur sa face frontale tournée vers l'ouverture (7) une ouverture de piston (26) par laquelle le premier piston (8) s'étend à l'intérieur du deuxième piston (9) et au niveau de laquelle le premier piston (8) est logé de manière mobile longitudinalement par rapport au deuxième piston (9) dans l'état de découplage.
